# FASCICULE DE BREVET EUROPEEN

(11) **EP 1 269 985 B1**
(45) Date de publication et mention de la délivrance du brevet: **30.11.2005**
(21) Numéro de dépôt: 02291385.9
(22) Date de dépôt: 05.06.2002
(51) Int. Cl.: A61K 7/48, A01N 43/90

(54) **Lingette et ses utilisations dans le domaine cosmétique**
Tüchlein und dessen Verwendungen im kosmetischen Bereich
Wipe and uses thereof in the cosmetics field

(30) Priorité: 22.06.2001 FR 0108284
(43) Date de publication de la demande: 02.01.2003
(73) Titulaire: L'OREAL, 75008 Paris (FR)
(72) Inventeur: Delambre, Patricia, 94480 Ablon-sur-Seine (FR); Touzan, Philippe, 75012 Paris (FR); Simon, Pascal, 94400 Vitry sur Seine (FR)
(74) Mandataire: Rasson, Catherine

(56) Documents cités:
- EP-A- 0 850 634
- WO-A-01/53443
- US-A- 5 141 803
- ALEXANDER P: "PRESERVATIVES IN PERSONAL CARE PRODUCTS" SOAP PERFUMERY AND COSMETICS, UNITED TRADE PRESS LTD. LONDON, GB, vol. 60, no. 3, 1 mars 1987 (1987-03-01), page 47-49 XP002039063 ISSN: 0037-749X
- MCCONVILLE J F: "QUATERNIUM-15 AS A COSMETIC PRESERVATIVE" DRUG AND COSMETIC INDUSTRY, XX, XX, mai 1986 (1986-05), pages 50-61, XP008002680

## Description

L'invention se rapporte à un article, plus particulièrement une lingette, comportant (A) un substrat insoluble dans l'eau, (B) une composition ajoutée ou imprégnée sur le substrat comprenant une phase aqueuse et du chlorure de N-(3-chloroallyl)-hexaminium, ainsi qu'aux utilisations dudit article dans le domaine cosmétique ou dermatologique, en particulier pour le nettoyage et/ou le démaquillage de la peau humaine, plus spécialement du visage, et des yeux.

Pour le nettoyage ou le démaquillage de la peau, il est connu d'utiliser des compositions du type lotion ou lait qui sont appliqués sur un support tel qu'un coton au moment de l'utilisation, et appliquées sur le visage.

Il est connu aussi d'utiliser des lingettes humides qui contiennent une composition d'imprégnation permettant le nettoyage ou le démaquillage de la peau, ce qui évite la manipulation et le transport de flacons contenant les lotions ou laits. Ces lingettes sont généralement constituées d'un substrat en une matière d'origine naturelle ou synthétique, de préférence en non tissé, imprégné d'une composition adaptée au but recherché, par exemple le nettoyage ou le démaquillage de la peau.

Les compositions d'imprégnation de ces lingettes contiennent généralement de l'eau et peuvent se présenter par exemple sous forme de solutions, de dispersions ou d'émulsions. Du fait de la présence d'eau dans ces compositions, il est nécessaire de les protéger contre le développement et la prolifération des micro-organismes en y introduisant des conservateurs. En effet, un tel développement de micro-organismes rendrait rapidement les compositions et par conséquent les articles les contenant, inaptes à l'utilisation.

Les conservateurs chimiques les plus couramment utilisés dans ce domaine sont notamment les parahydroxybenzoates d'alkyle en C₁-C₄ appelés ci-après parabens, et le phénoxyéthanol. Malheureusement, ces conservateurs présentent l'inconvénient de devoir être utilisés à des doses importantes pour apporter une protection anti-bactérienne efficace aux supports du type lingettes, et souvent à des doses plus importantes que dans le cas de laits et lotions démaquillantes présentées en flacons. Ces conservateurs en quantités plus importantes peuvent causer des intolérances sur la peau humaine, telles que irritations et/ou allergies, et plus spécialement sur les peaux sensibles.

On constate donc que subsiste le besoin d'un nouveau système conservateur ayant une efficacité antibactérienne sur les lingettes, au moins aussi efficace que les systèmes de l'art antérieur dans les mêmes gammes de concentration, mais ne présentant pas leurs inconvénients et notamment non irritants pour la peau et les yeux.

La demanderesse a trouvé, de manière surprenante, que l'utilisation du chlorure de N-(3-chloroallyl)-hexaminium ou chlorure de (chloro-3 allyl)-1 tri-azo-3,5,7 azonium-1 adamante (encore appelé en nom CTFA : quaternium-15), même en une quantité très faible, permettait d'obtenir une protection particulièrement efficace contre les microorganismes (bactéries, levures et moisissures) et d'obtenir une composition adaptée à l'imprégnation sur un support.

Certes, le Quaternium-15 est connu comme conservateur des compositions cosmétiques. Ainsi, le document EP-850634 décrit un système conservateur comprenant notamment du Quaternium-15. Par ailleurs, l'article de P. Alexander publié dans « Soap Perfumery and cosmetics », United Trade Press Ltd. London, vol. 60, n°3, 1 mars 1987 (1987-03-01), pages 47-49, et celui de J. F. McConville publié dans « Drug and Cosmetic Industry », mai 1986 (1986-05), pages 50-61, décrivent ce conservateur et des compositions le contenant.

En outre, le document WO-A-01/53443 décrit des lingettes antimicrobiennes contenant une composition antimicrobienne pouvant contenir entre autres, du chlorure de N-(3-chloroallyl)-hexaminium, et le document US-A-5,141,803 décrit des lingettes contenant un système conservateur à base de sorbate de potassium, d'acide citrique, de disodium EDTA et d'un biocide cationique choisi parmi le chlorure de polyhexaméthylène biguanide et le dichlorure de poly[(oxyéthylène(diméthylimino)éthylène(diméthylimino)éthylène).

Toutefois, la protection bactériologique d'une lingette pose des problèmes particuliers du fait que la surface de contact avec l'air est bien supérieure à celle d'un produit en pot ou en flacon, ce qui augmente le risque de contamination et donc oblige à une meilleure protection bactériologique. De plus, le support de la lingette peut être aussi une cause de contamination plus importante que la surface interne d'un flacon ou d'un pot. Le problème était donc d'avoir une bonne efficacité de protection bactériologique tout en ayant une faible quantité de conservateur pour avoir une moindre irritation.

Ainsi, la présente invention a pour objet une lingette pour la peau comportant (A) un substrat insoluble dans l'eau, non tissé, et (B) une composition ajoutée ou imprégnée sur le substrat, comprenant dans un milieu physiologiquement acceptable, une phase aqueuse et de 0,01 à 0,05 % en poids de chlorure de N-(3-chloroallyl)-hexaminium par rapport au poids total de la composition et de 0,005 à 0,3 % en poids de parahydroxy-benzoate d'alkyle en C₁-C₄.

Cet article (lingette) peut notamment être approprié pour le soin et/ou le traitement de la peau et notamment une lingette de nettoyage ou de démaquillage de la peau du visage et/ou du corps, et/ou une lingette de nettoyage ou de démaquillage des yeux, ainsi que pour le nettoyage des peaux à tendance grasse ou acnéique.

L'invention a donc aussi pour objet l'utilisation cosmétique de la lingette telle que définie ci-dessus, pour le nettoyage et/ou le démaquillage de la peau et/ou des yeux.

L'invention a encore pour objet l'utilisation cosmétique de 0,01 à 0,05 % en poids de chlorure de N-(3-chloroallyl)-hexaminium par rapport au poids total de la composition, en tant qu'agent de lutte contre les micro-organismes dans une composition cosmétique contenant une phase aqueuse et destinée à imprégner un substrat non tissé insoluble dans l'eau.

La lingette obtenue peut être utilisée par exemple pour traiter la peau grasse et/ou pour désinfecter la peau et la purifier en cas de présences de boutons à tendance acnéique, notamment si elle contient des agents antimicrobiens.

L'invention a encore pour objet l'utilisation cosmétique de la lingette telle que définie ci-dessus, pour le nettoyage des peaux à tendance grasse ou acnéique.

La composition utilisée selon l'invention comprend de 0,01 à 0,05 % et de préférence de 0,01 à 0,03 % en poids de Quaternium-15 par rapport au poids total de la composition.

Selon un mode préféré de réalisation de l'invention, la composition utilisée sur le substrat comprend aussi au moins un autre conservateur choisi parmi les sels de l'acide éthylène diamine tétracétique, et leurs mélanges.

Le parahydroxybenzoate d'alkyle en C₁-C₄ peut être choisi en particulier parmi le parahydroxybenzoate de méthyle, appelé ci-après méthylparaben, le parahydroxybenzoate d'éthyle, appelé ci-après éthylparaben et le parahydroxybenzoate de propyle, appelé ci-après propylparaben, et leurs mélanges. Selon un mode préféré de l'invention, il s'agit du méthylparaben ou du propylparaben. Le methylparaben est plus particulièrement préféré.

La composition utilisée selon l'invention peut comprendre par exemple de 0,01 à 0,3 % en poids, de préférence de 0,05 à 0,25 % en poids de parahydroxybenzoate d'alkyle, par rapport au poids total de la composition.

Le sel de l'acide éthylène diamine tétracétique peut être notamment un sel de métal alcalin et tout spécialement le sel de sodium. Selon un mode préféré de réalisation de l'invention, il s'agit du sel disodique de l'acide éthylène diamine tétracétique, du sel tétrasodique de l'acide éthylène diamine tétracétique ou de leurs mélanges.

La composition utilisée selon l'invention peut comprendre par exemple de 0,01 à 0,2 % en poids, et de préférence de 0,05 à 0,15 % en poids de sel de l'acide éthylène diamine tétracétique par rapport au poids total de la composition.

Le système conservateur comprenant le quaternium 15, ainsi que le parahydroxybenzoate d'alkyle en C₁-C₄ et/ou le sel de l'acide éthylène diamine tétracétique, représente généralement au total de 0,025 à 0,55 % en poids et mieux de 0,1 à 0,3 % en poids par rapport au poids total de la composition.

La composition utilisée selon l'invention pour l'imprégnation du substrat insoluble dans l'eau, contient un milieu aqueux physiologiquement acceptable, c'est-à-dire compatible avec la peau, les muqueuses, les cheveux et le cuir chevelu. Le milieu aqueux de la composition utilisée selon l'invention peut contenir, outre l'eau, un ou plusieurs solvants choisis parmi les alcools inférieurs comportant de 1 à 6 atomes de carbone, tels que l'éthanol ; les polyols tels que la glycérine ; les glycols comme le butylène glycol, l'isoprène glycol, l'hexylène glycol, le propylène glycol, les polyéthylène glycols tels que le PEG-8 ; le sorbitol ; les sucres tels que le glucose, le fructose, le maltose, le lactose, le sucrose ; et leurs mélanges.

Selon un mode préféré de réalisation de l'invention, la composition destinée à imprégner un support présente une viscosité de préférence inférieure à 150 mPa.s et plus préférentiellement inférieure à 100 mPa.s. Cette viscosité va de préférence de 1 mPa.s à 100 mPa.s, mesurée à la température ambiante (environ 25°C) avec un appareil RHEOMAT RM 180, mobile 1.

La composition utilisée selon l'invention a généralement un pH allant de 3 à 7,5 et de préférence de 6 à 7,5 et mieux de 6 à 7.

Les compositions selon l'invention peuvent se présenter sous toutes les formes galéniques appropriées pour une application topique, notamment sous forme de solutions aqueuses ou hydroalcooliques, de lotions, de laits, de gels aqueux ou hydroalcooliques, d'émulsions obtenues par dispersion d'une phase grasse dans une phase aqueuse (H/E) ou inversement (E/H), de suspensions, de microémulsions, de microcapsules, de microparticules, ou encore de dispersions vésiculaires de type ionique (liposomes) et/ou non ionique.

Selon un mode préféré de réalisation de l'invention, la composition se présente sous forme d'une émulsion H/E et constitue une lotion ou un lait.

Lorsque la composition utilisée selon l'invention est une émulsion, la proportion de la phase grasse (ou phase huileuse) peut aller de 5 à 80 % en poids, et de préférence de 5 à 50 % en poids par rapport au poids total de la composition. Les huiles, les émulsionnants et les coémulsionnants utilisés dans la composition sous forme d'émulsion sont choisis parmi ceux classiquement utilisés dans le domaine cosmétique ou dermatologique. L'émulsionnant et éventuellement le coémulsionnant sont présents, dans la composition, en une proportion allant de 0,3 à 30 % en poids, et de préférence de 0,5 à 20 % en poids par rapport au poids total de la composition.

La phase grasse ou phase huileuse contient habituellement au moins une huile. Comme huiles utilisables dans la composition de l'invention, on peut citer par exemple :
- les huiles hydrocarbonées d'origine animale, telles que le perhydrosqualène ;
- les huiles hydrocarbonées d'origine végétale, telles que les triglycérides liquides d'acides gras comportant de 4 à 10 atomes de carbone comme les triglycérides des acides heptanoïque ou octanoïque ou encore, par exemple les huiles d'amande douce, de tournesol, de maïs, de soja, de courge, de pépins de raisin, de sésame, de noisette, d'abricot, de macadamia, d'arara, de tournesol, de ricin, d'avocat, les triglycérides des acides caprylique/caprique comme ceux vendus par la société Stearineries Dubois ou ceux vendus sous les dénominations Miglyol 810, 812 et 818 par la société Dynamit Nobel, l'huile de jojoba, l'huile de beurre de karité ;
- les esters et les éthers de synthèse, notamment d'acides gras, comme les huiles de formules R¹COOR² et R¹OR² dans laquelle R¹ représente le reste d'un acide gras comportant de 8 à 29 atomes de carbone, et R² représente une chaîne hydrocarbonée, ramifiée ou non, contenant de 3 à 30 atomes de carbone, comme par exemple l'huile de Purcellin, l'isononanoate d'isononyle, le myristate d'isopropyle, le palmitate d'isopropyle, le palmitate d'éthyl-2-hexyle (ou palmitate d'octyle), le stéarate d'octyl-2-dodécyle, l'érucate d'octyl-2-dodécyle, l'isostéarate d'isostéaryle ; les esters hydroxylés comme l'isostéaryl lactate, l'octylhydroxystéarate, l'hydroxystéarate d'octyldodécyle, le diisostéaryl-malate, le citrate de triisocétyle, les heptanoates, octanoates, décanoates d'alcools gras ; les esters de polyol, comme le dioctanoate de propylène glycol, le diheptanoate de néopentylglycol et le diisononanoate de diéthylèneglycol ; et les esters du pentaérythritol comme le tétraisostéarate de pentaérythrityle,
- les hydrocarbures linéaires ou ramifiés, d'origine minérale ou synthétique, tels que les huiles de paraffine, volatiles ou non, et leurs dérivés, la vaseline, les polydécènes, le polyisobutène hydrogéné tel que l'huile de parléam ;
- les alcools gras ayant de 8 à 26 atomes de carbone, comme l'alcool cétylique, l'alcool stéarylique et leur mélange (alcool cétéarylique), l'octyldodécanol, le 2-butyloctanol, le 2-hexyldécanol, le 2-undécylpentadécanol, l'alcool oléique ou l'alcool linoléique ;
- les alcools gras alcoxylés et notamment éthoxylés tels que l'oleth-12 ou le ceteareth-20 ;
- les huiles fluorées partiellement hydrocarbonées et/ou siliconées comme celles décrites dans le document JP-A-2-295912. Comme huiles fluorées, on peut citer aussi le perfluorométhylcyclopentane et le perfluoro-1,3 diméthylcyclohexane, vendus sous les dénominations de "FLUTEC PC1®" et "FLUTEC PC3®" par la Société BNFL Fluorochemicals; le perfluoro-1,2-diméthylcyclobutane; les perfluoroalcanes tels que le dodécafluoropentane et le tétradécafluorohexane, vendus sous les dénominations de "PF 5050®" et "PF 5060®" par la Société 3M, ou encore le bromoperfluorooctyle vendu sous la dénomination "FORALKYL®" par la Société Atochem; le nonafluorométhoxybutane vendu sous la dénomination "MSX 4518®" par la Société 3M et le nonafluoréthoxyisobutane ; les dérivés de perfluoromorpholine, tels que la 4-trifluorométhyl perfluoromorpholine vendue sous la dénomination "PF 5052®" par la Société 3M ;
- les huiles de silicone comme les polyméthylsiloxanes (PDMS) volatiles ou non à chaîne siliconée linéaire ou cyclique, liquides ou pâteux à température ambiante, notamment les cyclopolydiméthylsiloxanes (cyclométhicones) telles que la cyclohexasiloxane ; les polydiméthylsiloxanes comportant des groupements alkyle, alcoxy ou phényle, pendant ou en bout de chaîne siliconée, groupements ayant de 2 à 24 atomes de carbone ; les silicones phénylées comme les phényltriméthicones, les phényldiméthicones, les phényltriméthylsiloxydiphénylsiloxanes, les diphényl-diméthicones, les diphénylméthyldiphényl trisiloxanes, les 2-phényléthyltriméthyl-siloxysilicates, et les polyméthylphénylsiloxanes ;
- leurs mélanges.

On entend par "huile hydrocarbonée" dans la liste des huiles citées ci-dessus, toute huile comportant majoritairement des atomes de carbone et d'hydrogène, et éventuellement des groupements ester, éther, fluoré, acide carboxylique et/ou alcool.

La composition selon l'invention peut contenir des émulsionnants ioniques ou non ioniques, dont le choix dépend de l'émulsion souhaitée (E/H ou H/E). On peut utiliser comme émulsionnants ceux habituellement utilisés dans le domaine considéré.

Comme émulsionnants, on peut citer par exemple les tensioactifs non ioniques tels que les esters d'acide gras et de polyols, et leurs dérivés oxyalkylénés et notamment oxyéthylénés ; les éthers d'alcools gras et de polyols, et leurs dérivés oxyalkylénés et notamment oxyéthylénés, et leurs mélanges. Quand il s'agit d'esters d'acide gras et de polyols oxyalkylénés ou d'éthers d'alcools gras et de polyols oxyalkylénés, il peut y avoir par exemple de 1 à 150 groupes oxyalkylénés et notamment oxyéthylénés. Comme émulsionnants, on peut citer plus particulièrement le mélange de stéarate de glycéryle et de stéarate de PEG-100, vendu sous le nom d'Arlacel 165 par la société ICI ; les éthers d'alcools gras polyoxyéthylénés comportant de 1 à 100 groupes oxyéthylénés, comme par exemple le ceteareth-12 et le ceteareth-20, ainsi que les mélanges les contenant comme le mélange commercialisé sous la dénomination Emulgade CM par la société Henkel (mélange de isononanoate de cétéaryle, ceteareth-20, alcool cétéarylique, stearate de glyceryle, glycérine, ceteareth-12 et palmitate de cétyle). Les émulsionnants cités ci-dessus sont utilisés pour la préparation d'émulsions H/E.

On peut ajouter aussi dans la composition de invention des tensioactifs non ioniques, anioniques, amphotères ou zwitterioniques, favorisant l'élimination du maquillage et des impuretés, et pouvant rendre la composition moussante. Il peut s'agir notamment de tensioactifs moussants. Comme tensioactifs de ce type, on peut citer par exemple :
(1) parmi les tensioactifs non ioniques, les polymères blocs oxyéthylénés oxypropylénés tels que le Poloxamer 184 (nom CTFA) ; les alkylpolyglycosides et notamment les alkylpolyglucosides (APG) ayant un groupe alkyle comportant de 6 à 30 atomes de carbone (alkyl-C₆-C₃₀ polyglucosides) et de préférence 8 à 16 atomes de carbone, comme par exemple le decylglucoside (Alkyl-C9/C11-polyglucoside (1.4) tel que le produit commercialisé sous la dénomination MYDOL 10 par la société Kao Chemicals, le produit commercialisé sous la dénomination PLANTAREN 2000 UP ou PLANTACARE 2000 UP par la société Henkel, et le produit commercialisé sous la dénomination ORAMIX NS 10 par la société Seppic ; le caprylyl/capryl glucoside comme le produit commercialisé sous la dénomination ORAMIX CG 110 par la Société Seppic ; le laurylglucoside comme les produits commercialisés sous les dénominations PLANTAREN 1200 N et PLANTACARE 1200 par la société Henkel ; et le coco-glucoside comme le produit commercialisé sous la dénomination PLANTACARE 818/UP par la société Henkel ;
(2) parmi les tensioactifs anioniques, les alkylsulfates, les alkyl éther sulfates et leurs sels, notamment leurs sels de sodium, comme le mélange de Sodium Laureth Sulfate / Magnesium Laureth Sulfate / Sodium Laureth-8 Sulfate /Magnesium Laureth-8 Sulfate, vendu sous le nom de Texapon ASV par la société Henkel ; le lauryl éther sulfate de sodium (C12-14 70/30) (2,2 OE) commercialisé sous les dénominations SIPON AOS 225 ou TEXAPON N702 PATE par la société Henkel, le lauryl éther sulfate d'ammonium (C12-14 70/30) (3 OE) commercialisé sous la dénomination SIPON LEA 370 par la société Henkel ; l'alkyl (C12-C14) éther (9 OE) sulfate d'ammonium commercialisé sous la dénomination RHODAPEX AB/20 par la société Rhodia Chimie ;
(3) parmi les tensioactifs amphotères ou zwitterioniques, les dérivés alkylamido alkylamines tels que le N-cocoyl-N-carboxyméthoxyéthyl-N-carboxyméthyléthylènediamine N-di-sodique (nom CTFA : Disodium cocoampho-diacetate) commercialisé en solution aqueuse saline sous la dénomination MIRANOL C2M CONC NP par la société Rhodia Chimie; le N-cocoyl-N-hydroxyéthyl-N-carboxyméthyl-éthylènediamine N-sodique (nom CTFA: sodium cocamphoacetate) et le mélange d'éthanolamides d'acide de coco (nom CTFA Cocamide DEA).

La composition peut comprendre aussi un mélange de ces tensioactifs.

La composition utilisée pour imprégner le substrat peut comprendre en outre les adjuvants classiquement mis en oeuvre dans les domaines considérés, comme par exemple les solvants organiques, les agents solubilisants, les agents épaississants et gélifiants hydrophiles ou lipophiles, les adoucissants, les antioxydants, les opacifiants, les agents stabilisants, les agents moussants, les charges, les chélateurs, les parfums, les filtres, les huiles essentielles, les matières colorantes, les pigments, les actifs hydrophiles ou lipophiles, les vésicules lipidiques encapsulant éventuellement un ou plusieurs actifs, ou tout autre ingrédient habituellement utilisé en cosmétique ou dermatologie. Elle peut éventuellement contenir aussi des conservateurs autres que ceux cités ci-dessus. Les quantités des différents constituants des compositions selon l'invention sont celles classiquement utilisées dans les domaines considérés. Bien entendu, ces adjuvants doivent être de nature et utilisés en quantité telle qu'ils ne perturbent pas le système conservateur selon l'invention. La quantité de ces adjuvants peut aller par exemple de 0,01 à 30 % en poids par rapport au poids total de la composition.

Comme actifs, on peut citer par exemple, les actifs antiséborrhéiques qui permettent un nettoyage de l'excédent de sebum sur la peau, et les agents antimicrobiens qui éliminent de la peau les microorganismes qui y sont éventuellement présents, et leurs mélanges.

Comme actifs antiséborrhéiques, on peut citer par exemple le soufre et les dérivés soufrés, le peroxyde de benzoyle, les dérivés de zinc tels que le sulfate de zinc et l'oxyde de zinc, le chlorure d'aluminium, le disulfure de sélénium, les vitamines B et notamment le panthénol (vitamine B5) et la niacinamide (vitamine B6 ou PP), et leurs mélanges.

Comme antimicrobiens, on peut citer par exemple les actifs suivants : dérivés de β-lactam, dérivés de quinolone, ciprofloxacine, norfloxacine, tétracycline et ses sels (chlorhydrate), érythromycine et ses sels (de zinc, estolate, stéarate), amikacine et ses sels (sulfate), 2,4,4'-trichloro-2'-hydroxy diphenyl éther (triclosan), 3,4,4'-trichlorobanilide (tricarban), phénoxyéthanol, phénoxypropanol, phénoxyisopropanol, doxycycline et ses sels (chlorhydrate), capréomycine et ses sels (sulfate), chlorhexidine et ses sels (gluconate, chlorhydrate), chlorotétracycline et ses sels (chlorhydrate), oxytétracycline et ses sels (chlorhydrate), clindamycine et ses sels (chlorhydrate), éthambutol et ses sels (chlorhydrate), hexamidine et ses sels (iséthionate), métronidazole et ses sels (chlorhydrate), pentamidine et ses sels (chlorhydrate), gentamicine et ses sels (sulfate), kanamycine et ses sels (sulfate), linéomycine et ses sels (chlorhydrate), méthacycline et ses sels (chlorhydrate), méthenamine et ses sels (hippurate, mandelate), minocycline et ses sels (chlorhydrate), néomycine et ses sels (sulfate), netilmicine et ses sels (sulfate), paromomycine et ses sels (sulfate), streptomycine et ses sels (sulfate), tobramycine et ses sels (sulfate), miconazole et ses sels (chlorhydrate), amanfadine et ses sels (sulfate, chlorhydrate), octopirox, parachlorometaxylenol, nystatine, tolnaftate, zinc pyrithione, clotrimazole, acide salicylique, acide n-octanoyl-5 salicylique (ou acide capryloylsalicylique), peroxyde de benzoyle, acide 3-hydroxybenzoique, acide glycolique, acide lactique, acide 4-hydroxy-benzoique, acide acétylsalicylique, acide 2-hydroxybutanoique, acide 2-hydroxypentanoique, acide 2-hydroxyhexanoique, acide phytique , acide N-acetyl-L-cysteine, acide lipoique, acide azélaïque, acide arachidonique, ibuprofen, naproxen, hydrocortisone, acetominophen, resorcinol, chlorhydrate de lidocaine, sulfate de néocycine, octoxyglycérine, octanoylglycine (ou capryloylglycine), caprylylglycol (1,2-octanediol), acide 10-hydroxy-2-décanoique, et leurs mélanges. Les agents antimicrobiens préférés sont le 2,4,4'-trichloro-2'-hydroxy diphenyl éther, le 3,4,4'-trichlorobanilide, le phénoxyéthanol, le phénoxypropanol, le phénoxyisopropanol, la chlorhexidine et ses sels, l'octopirox, le zinc pyrithione, l'acide salicylique, l'acide n-octanoyl-5 salicylique, le peroxyde de benzoyle, l'acide 3-hydroxybenzoique, l'acide glycolique, l'acide lactique, l'acide 4-hydroxy-benzoique, l'acide acétylsalicylique, l'acide 2-hydroxybutanoique, l'acide 2-hydroxypentanoique, l'acide 2-hydroxyhexanoique, l'acide phytique , l'acide N-acetyl-L-cysteine, l'acide lipoique, l'acide azélaïque, l'acide arachidonique, l'octoxyglycérine, l'octanoylglycine, le caprylylglycol, l'acide 10-hydroxy-2-décanoique, et leurs mélanges.

Comme gélifiants hydrophiles, on peut citer en particulier les polymères carboxyvinyliques comme les carbomer ; les copolymères acryliques modifiés tels que les copolymères d'acrylates/alkylacrylates comme les produits commercialisés sous les dénominations Pemulen par la société Goodrich ; les polyacrylamides comme le produit commercialisé sous la dénomination Sepigel 305 par la société Seppic, ou le poly(acide 2-acrylamido 2-méthylpropane sulfonique) commercialisé par la société Hoechst sous la dénomination commerciale « Hostacerin AMPS » (nom CTFA : ammonium polyacryldimethyltauramide) ; les polysaccharides, notamment les dérivés cellulosiques et les gommes naturelles comme lagomme de xanthane ou la gomme guar ; et les argiles. Comme gélifiants lipophiles, on peut citer les argiles modifiées comme les bentones, les sels métalliques d'acides gras, la silice hydrophobe, les polyéthylènes, et leurs mélanges. On peut aussi utiliser comme gélifiants, les polymères à fonction hydrophobe, tels que les polysaccharides à chaîne hydrophobe comme les gommes de guar quaternisées.

La composition selon l'invention est avantageusement exempte d'acide 2-pyrrolidone 5-carboxylique.

Les compositions selon l'invention sont préparées selon les techniques bien connues de l'homme de l'art du domaine.

Selon un mode particulier de réalisation de l'invention, la composition se présente sous forme d'une émulsion PIT. Cette technique d'obtention d'émulsion H/E est, dans son principe, bien connue de l'homme de l'art et est notamment décrite dans les articles "Phase Inversion Emusification", par Th. Förster et al, paru dans Cosmetics & Toiletries, vol. 106, Decembre 1991, pp 49-52, «application of the phase-inversion-température method to the emulsification of cosmetics» par T. MITSUI et al, paru dans American Cosmetics and Perumery, vol 87, Decembre 1972. Son principe est le suivant : on prépare une émulsion E/H (introduction de l'eau dans l'huile) à une température qui doit être supérieure à la température d'inversion de phase (en Anglais : Phase Inversion Temperature ou PIT) du système, c'est à dire la température à laquelle l'équilibre entre les propriétés hydrophiles et lipophiles du ou des émulsionnants mis en oeuvre est atteint ; à température élevée (>PIT), l'émulsion est de type eau-dans-huile, et au cours de son refroidissement, à la température d'inversion de phase, cette émulsion s'inverse pour devenir une émulsion de type cette fois huile-dans-eau, et ceci en étant passée auparavant par un état de microémulsion. Cette technique permet d'obtenir des émulsions H/E dites "ultrafines", dans lesquelles la taille moyenne des globules constituant la phase grasse est comprise dans des limites bien déterminées, à savoir entre 50 et 1000 nm. Ces émulsions sont extrêmement fluides et sont particulièrement bien appropriées pour imprégner des substrats insolubles dans l'eau afin de constituer des articles ou lingettes de nettoyage. Selon un mode particulier de réalisation, l'émulsion PIT est préparée sous forme concentrée puis diluée, généralement juste avant l'imprégnation, par une à neuf parties d'une phase aqueuse pouvant également contenir tout ou une partie des conservateurs préalablement dissous.

Le substrat insoluble dans l'eau est choisi dans le groupe des matériaux non-tissés. Il peut s'agir notamment d'un substrat non tissé à base de fibres d'origine naturelle (lin, laine, coton, soie) ou d'origine synthétique (dérivés de cellulose, viscose, dérivés polyvinyliques, polyesters comme téréphtalate de polyéthylène, polyoléfines comme polyéthylène ou polypropylène, polyamides comme le Nylon, dérivés acryliques). Les non tissés sont décrits de façon générale dans RIEDEL « Nonwoven Bonding Methods & Materials », Nonwoven World (1987). Ces substrats sont obtenus selon les procédés usuels de la technique de préparation des non-tissés. Selon un mode particulier de réalisation de l'invention, le substrat insoluble peut contenir un au moins des conservateurs de l'invention, fixés au support par les moyens connus de greffage des agents biocides sur les fibres.

Ce substrat peut comporter une ou plusieurs couches ayant des propriétés identiques ou différentes et avoir des propriétés d'élasticité, de douceur et autres appropriées à l'usage recherché. Les substrats peuvent comporter par exemple deux parties ayant des propriétés d'élasticité différentes comme décrit dans le document WO-A-99/13861 ou comporter une seule couche avec des densités différentes comme décrit dans le document WO-A-99/25318 ou comporter deux couches de textures différentes comme décrit dans le document WO-A-98/18441.

Le substrat peut avoir toute taille et toute forme appropriées au but recherché. Il a généralement une surface comprise entre 0,005 m² et 0,1 m², de préférence entre 0,01 m² et 0,05 m². Il se présente de préférence sous la forme de lingettes rectangulaires ou de compresses rondes.

L'article final comportant le substrat et la composition d'imprégnation est généralement à l'état humide, avec un taux d'imprégnation de la composition allant par exemple de 200 à 1000 %, et de préférence de 250 à 350 % en poids de composition par rapport au poids de substrat. Les techniques d'imprégnation des substrats par des compositions sont bien connues dans ce domaine et sont toutes applicables à la présente invention. En général, la composition d'imprégnation est ajoutée au substrat par une ou plusieurs techniques comprenant l'immersion, l'enduction, la vaporisation, etc.

Il est aussi possible de constituer une lingette présentée à l'état sec soit en éliminant l'eau de la composition après son imprégnation sur le substrat, soit en imprégnant le substrat avec une composition sous forme sèche à l'état de poudre, granule ou film, par tous les moyens de réalisation connus comme le soudage et le collage de multicouches par voies thermiques ou ultrasons. Dans ce dernier mode de réalisation, la composition est séchée par tout moyen connu : atomisation, lyophylisation ou autre procédé analogue.

On peut ainsi obtenir selon l'utilisation envisagée, des lingettes humides ou des lingettes sèches. Les lingettes humides peuvent être utilisées telles quelles alors que les lingettes sèches sont mouillées avant utilisation.

L'invention a aussi pour objet un procédé cosmétique de nettoyage et/ou de démaquillage de la peau et/ou des yeux, consistant à passer sur la peau et/ou les yeux, un article tel que défini ci-dessus.

Dans tout ce qui suit, les pourcentages sont donnés en poids sauf mention contraire.

D'autre caractéristiques et avantages de l'invention pourront apparaître dans les exemples qui suivent, qui sont donnés à titre purement illustratif et nullement limitatif.

### Test

Le test suivant met en évidence l'activité de l'association selon invention sur les micro-organismes.

Les étapes pour réaliser ce test sont les suivantes :

### ■ 1^{ère} étape : sur le jus seul

1) Culture du micro-organisme des souches utilisées :
   *Enterococcus faecalis* (bactérie)
   *Candida albicans* (levure)
   *Aspergillus niger* (moisissure)

   Les souches sont préparées dans le milieu de culture tryptone-sel.
2) Préparation de l'inoculum : L'inoculum est constitué d'une culture de 24 heures du germe-test sur un milieu nutritif gélosé approprié et dilué de façon à obtenir une suspension contenant 10⁸ germes/ml. Un dénombrement de 5 inocula est effectué.
3) Préparation de l'échantillon : On dépose dans 5 flacons de verre appelés piluliers, 20 g du produit à tester.
4) Inoculation : on introduit 0,2 ml d'inoculum de chaque souche dans un pilulier différent et on homogénéise. On obtient donc une contamination à 10⁶ germes/g. Les piluliers sont alors mis à incuber à 22°C pendant 7 jours.
5) Prélèvements et dénombrements :
   Après les 7 jours d'incubation, 1 g de produit est prélevé de chacun des piluliers. Des dilutions décimales sont effectuées dans un diluant renfermant des inhibiteurs de conservateurs qui vont stopper l'action des conservateurs. Ces dilutions sont étalées en surface de boites de Pétri gélosées, mises ensuite à incuber à 35°C. Le dénombrement est effectué à 7 jours.

Le nombre de germes doit être inférieur à 100 pour que le système conservateur soit satisfaisant.

### 2^{ère} étape : sur les lingettes imprégnées

Inoculation de 250 µl des cinq souches suivantes :

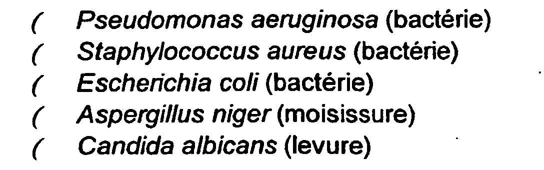

Les compositions dont le dénombrement à 7 jours est inférieur ou égal à celui du témoin pour l'ensemble des germes testés sont considérées comme satisfaisantes et notées « conforme ». Les autres formules sont notées comme « non conformes ».

Les associations suivantes ont été testées :

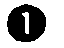 **Association 1 selon l'invention**

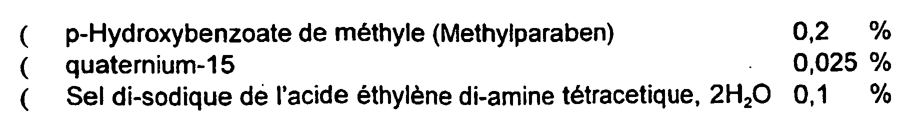

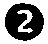 **Association 2 (exemple** **comparatif)**

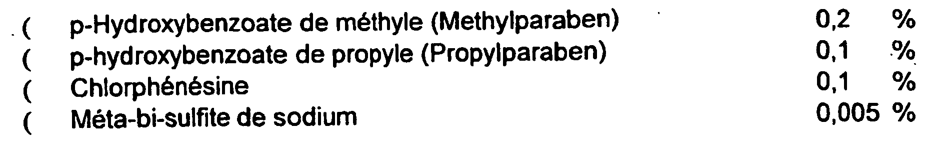

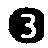 **Association 3 (exemple comparatif)**

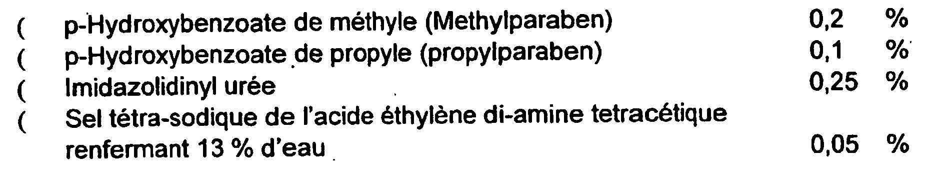

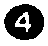 **Association 4 (exemple comparatif)**

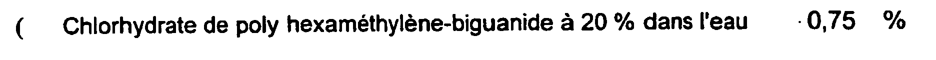

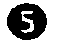 **Association 5 (exemple comparatif)**

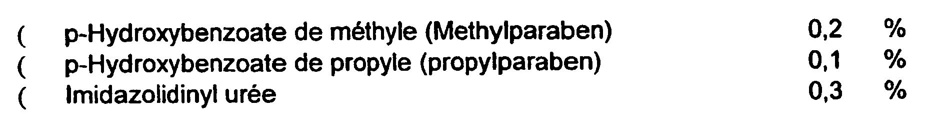

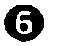 **Association 6 (exemple comparatif)**

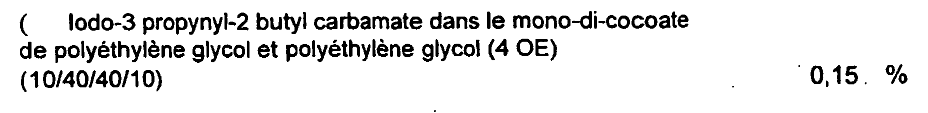

Dans notre étude bactériologique, nous avons utilisé comme référence, le système conservateur suivant :

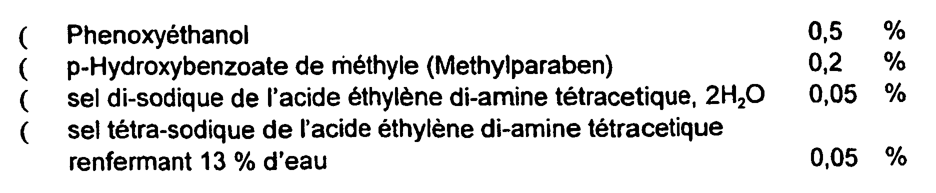

Les résultats obtenus sont rassemblés dans le tableau 1 suivant :

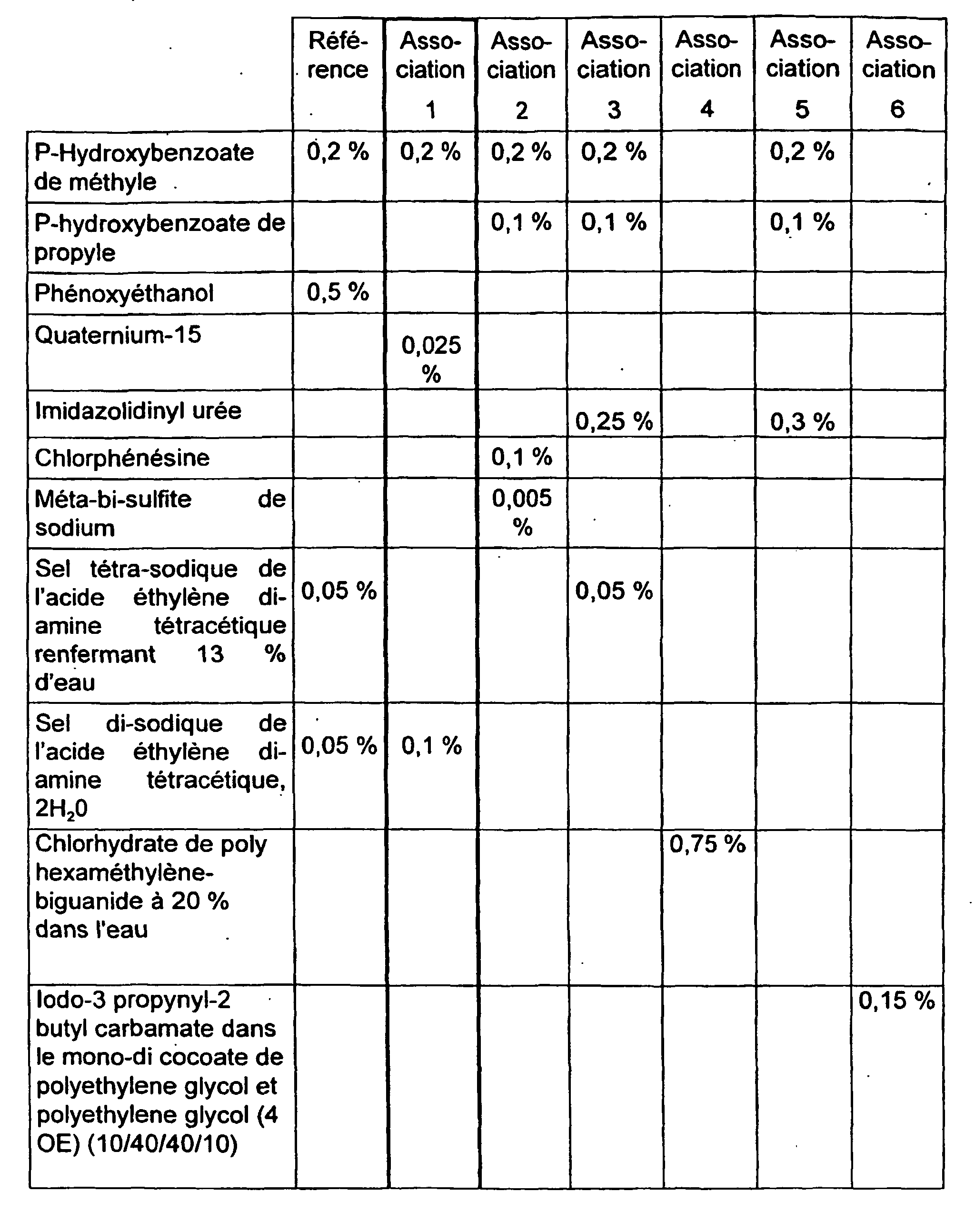

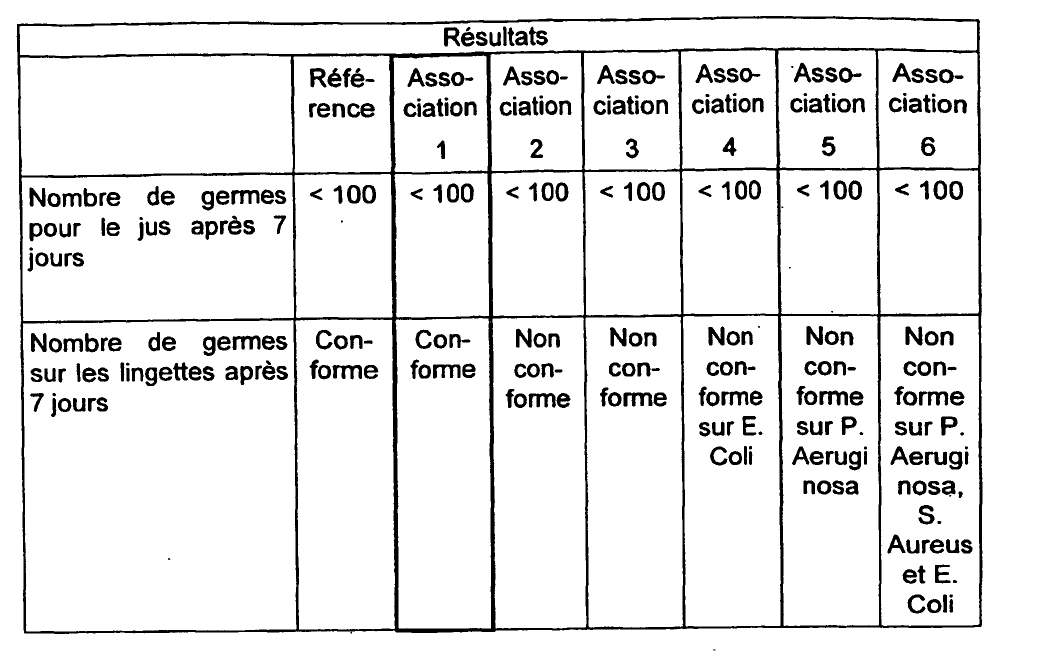

Les résultats montrent que seul le système conforme à l'invention donne des résultats équivalents à la référence, tout en donnant des produits ne posant aucun problème d'innocuité.

### Exemples de composition

Les exemples ci-après de compositions selon invention sont donnés à titre d'illustration. Les quantités y sont données en % en poids, sauf mention contraire.

### Exemple 1 : lait démaquillant (Emulsion H/E)

*Phase A :*
   - Stéarate de glycérol / stéarate de PEG 100 (Arlacel 165) 0,6 %
   - Alcool cétylique 0,15 %
   - Parfum qs %
*Phase B*
   - Methylparaben 0,2 %
   - Sel di-sodique de l'acide éthylène di-amine tétracétique, 2H₂O 0,1 %
   - Eau déminéralisée qs 100 %
*Phase C :*
   - Carbomer 0,1 %
   - Gomme de xanthane 0,1 %
   - Palmitate d'isopropyle 5 %
*Phase D*
   - Triéthanolamine 0,1 %
*Phase E :*
   - Quaternium 15 0,025 %
   - Eau déminéralisée 30 %

Mode opératoire : on fait chauffer séparément les phases A et B à 75/80°C. On ajoute la phase A dans la phase B sous agitation, puis on maintient cette agitation pendant 5 minutes. On ajoute ensuite les phases C et D, puis on fait refroidir sous agitation. A 30 °C, on ajoute la phase E. On refroidit à la température ambiante (environ 25 °C), et on obtient un lait.

Les lingettes imprégnées avec ce lait démaquillant permettent de démaquiller le visage et les yeux sans irritation, tout en ayant une très bonne conservation antibactérienne.

### Exemple 2 : lait démaquillant (Emulsion PIT)

*Phase A :*
   - Isononanoate de cétéaryle 1,8 %
   - Alcool cétéarylique 0,75 %
   - Alcool cétéarylique oxyéthyléné (20 OE) (ceteareth-20) 0,75 %
   - Alcool cétéarylique oxyéthyléné (12 OE) (ceteareth-12) 0,05 %
   - Glycérine 0,25 %
   - Stéarate de glycéryle 0,25 %
   - Acide benzoïque 0,1 %
   - Palmitate de cétyle 0,05 %
   - Parfum 0,1 %
   - Eau déminéralisée 6 %
*Phase B*
   - Alkyl (C8/10/12/14/16) polyglucoside (1.4) en solution aqueuse à 53 % vendu sous le nom Plantacare 2000 par Cognis 0,1 %
   - Méthylparaben 0,2 %
   - Sel di-sodique de l'acide éthylène di-amine tétracétique, 2H₂O 0,1 %
   - Quaternium-15 0,025 %
   - Eau déminéralisée qsp 100 %

Mode opératoire: La phase A est préparée par les moyens connus pour émulsifier selon le procédé de température d'inversion de phase (technologie PIT). Après retour à température ambiante, la phase B est introduite sous agitation douce non cisaillante. Selon un mode préféré de préparation, on utilise pour préparer la phase A, le mélange de isononanoate de cétéaryle, ceteareth-20, alcool cétéarylique, stéarate de glyceryle, glycérine, ceteareth-12 et palmitate de cétyle, commercialisé sous le nom de Emulgade CM par la société Cognis. Après l'introduction du parfum à température ambiante dans ce mélange, la phase B est introduite juste avant l'imprégnation des supports.

### Exemple 3 : lotion démaquillante (lotion aqueuse)

- Disodium cocoamphodiacetate (MIRANOL C2M CONC NP de Rhodia Chimie) 1,2 %
- Hexylène glycol 1 %
- Methylparaben 0,1 %
- EDTA disodique 0,1 %
- Quaternium 15 0,02 %
- Triéthanolamine 0,08 %
- Eau déminéralisée qsp 100 %

Mode opératoire : on fait chauffer l'eau déminéralisée à 80 °C puis on ajoute successivement sous agitation, le Disodium cocoamphodiacetate, l'hexylène glycol, le methylparaben, puis l'EDTA disodique. On refroidit à 40 °C sous agitation. A cette température, on ajoute le Quaternium 15 puis la triéthanolamine. On refroidit à 25 °C et on obtient la lotion.

Un non tissé en viscose/Polyester 70/30, de grammage 65 g/m2 est imprégné avec 325 % en poids de composition par rapport au poids de non tissé, par les compositions des exemples 1 à 3, puis coupé sous la forme de lingettes rectangulaires de dimensions de 150 cm x 200 cm.

Une mousse polyuréthane peut être également imprégnée, par exemple à 100 % en poids de composition par rapport au poids de mousse, par les compositions des exemples 1 à 3. Elle se présentera avantageusement sous la forme d'une compresse ronde de diamètre 70 mm et d'épaisseur 1,5 mm.

## Revendications

1. Lingette pour la peau comportant (A) un substrat insoluble dans l'eau, non tissé, et (B) une composition ajoutée ou imprégnée sur le substrat, comprenant dans un milieu physiologiquement acceptable, une phase aqueuse et de 0,01 à 0,05 % en poids de chlorure de N-(3-chloroallyl)-hexaminium par rapport au poids total de la composition et de 0,005 à 0,3 % en poids de parahydroxy-benzoate d'alkyle en C₁-C₄.

2. Lingette selon la revendication 1, **caractérisée en ce que** la composition comprend de 0,01 à 0,03 % en poids de chlorure de N-(3-chloroallyl)-hexaminium par rapport au poids total de la composition.

3. Lingette selon la revendication 1 ou 2, **caractérisée en ce que** la composition comprend en outre au moins un conservateur choisi parmi les sels de l'acide éthylène diamine tétracétique, et leurs mélanges.

4. Lingette selon l'une quelconque des revendications précédentes, **caractérisée en ce que** le parahydroxybenzoate d'alkyle en C₁-C₄ est choisi parmi le parahydroxybenzoate de méthyle, le parahydroxybenzoate d'éthyle, le parahydroxybenzoate de propyle et leurs mélanges.

5. Lingette selon la revendication précédente, **caractérisée en ce que** le parahydroxybenzoate est le parahydroxybenzoate de méthyle.

6. Lingette selon la revendication 3, **caractérisée en ce que** le sel de l'acide éthylène diamine tétracétique est choisi parmi le sel disodique de l'acide éthylène diamine tétracétique, le sel tétrasodique de l'acide éthylène diamine tétracétique et de leurs mélanges.

7. Lingette selon l'une quelconque des revendications 3 ou 6, **caractérisée en ce que** la composition comprend de 0,01 à 0,2 % en poids de sel de l'acide éthylène diamine tétracétique par rapport au poids total de la composition.

8. Lingette selon l'une quelconque des revendications précédentes, **caractérisée en ce que** la composition présente une viscosité inférieure à 100 mPa.s.

9. Lingette selon l'une quelconque des revendications précédentes, **caractérisée en ce que** la composition se présente sous forme d'une solution aqueuse ou hydroalcoolique, d'une émulsion, d'une microémulsion, d'un gel aqueux ou hydroalcoolique, d'une dispersion de vésicules.

10. Lingette selon l'une quelconque des revendications précédentes, **caractérisée en ce que** la composition se présente sous forme d'une émulsion H/E.

11. Lingette selon l'une quelconque des revendications précédentes, **caractérisée en ce que** la composition se présente sous forme d'une émulsion PIT.

12. Lingette selon l'une quelconque des revendications précédentes, **caractérisée en ce que** la composition comprend en outre un émulsionnant choisi parmi les esters d'acide gras et de polyols ; les esters d'acide gras et de polyols oxyalkylénés ; les éthers d'alcools gras et de polyols ; les éthers d'alcools gras et de polyols oxyalkylénés ; et leurs mélanges.

13. Lingette selon l'une quelconque des revendications précédentes, **caractérisée en ce que** la composition comprend, en outre, au moins un adjuvant choisi parmi les solvants organiques, les agents solubilisants, les agents épaississants et gélifiants, les adoucissants, les antioxydants, les opacifiants, les agents stabilisants, les agents moussants, les charges, les chélateurs, les parfums, les filtres, les huiles essentielles, les matières colorantes, les pigments, les actifs, les vésicules lipidiques.

14. Lingette selon la revendication précédente, **caractérisée en ce que** l'actif est choisi parmi les actifs antiséborrhéiques, les agents antimicrobiens et leurs mélanges.

15. Lingette selon la revendication précédente, **caractérisée en ce que** l'actif antiséborrhéique est choisi parmi le soufre et les dérivés soufrés, le peroxyde de benzoyle, les dérivés de zinc, le chlorure d'aluminium, le disulfure de sélénium, le panthénol, la niacinamide, et leurs mélanges.

16. Lingette selon la revendication 14 ou 15, **caractérisée en ce que** l'actif antimicrobien est choisi parmi le 2,4,4'-trichloro-2'-hydroxy diphenyl éther, le 3,4,4'-trichlorobanilide, le phénoxyéthanol, le phénoxypropanol, le phénoxyisopropanol, la chlorhexidine et ses sels, l'octopirox, le zinc pyrithione, l'acide salicylique, l'acide n-octanoyl-5 salicylique, le peroxyde de benzoyle, l'acide 3-hydroxybenzoique, l'acide glycolique, l'acide lactique, l'acide 4-hydroxybenzoique, l'acide acétylsalicylique, l'acide 2-hydroxybutanoique, l'acide 2-hydroxypentanoique, l'acide 2-hydroxyhexanoique, l'acide phytique, l'acide N-acetyl-L-cysteine, l'acide lipoique, l'acide azélaïque, l'acide arachidonique, l'octoxyglycérine, l'octanoylglycine, le caprylylglycol, l'acide 10-hydroxy-2-décanoique, et leurs mélanges.

17. Lingette selon l'une quelconque des revendications précédentes, **caractérisée en ce que** la composition comprend, en outre, au moins un tensioactif moussant.

18. Lingette selon la revendication précédente, **caractérisée en ce que** le tensioactif moussant est choisi parmi les polymères blocs oxyéthylénés oxypropylénés, les alkylpolyglucosides ; les alkylsulfates, les alkyl éther sulfates et leurs sels ; les dérivés alkylamido alkylamines ; et leurs mélanges.

19. Lingette selon l'une quelconque des revendications précédentes, **caractérisée en ce que** la composition est exempte d'acide 2-pyrrolidone 5-carboxylique.

20. Lingette selon l'une quelconque des revendications précédentes, **caractérisée en ce que** la composition a un pH de 6 à 7,5.

21. Lingette selon l'une quelconque des revendications précédentes, **caractérisée en ce que** la composition se présente sous forme d'une lotion ou d'un lait.

22. Lingette selon l'une quelconque des revendications précédentes, **caractérisée en ce qu'**elle comprend de 200 à 1000 % en poids de composition par rapport au poids de substrat.

23. Lingette selon l'une quelconque des revendications précédentes, **caractérisée en ce qu'**elle constitue une lingette de soin et/ou de traitement de la peau.

24. Lingette selon l'une quelconque des revendications précédentes, **caractérisée en ce qu'**elle constitue une lingette de nettoyage et/ou de démaquillage de la peau et/ou des yeux.

25. Utilisation cosmétique de la lingette selon l'une quelconque des revendications 1 à 22, pour le nettoyage et/ou le démaquillage de la peau et/ou des yeux.

26. Utilisation cosmétique d'une lingette selon l'une quelconque des revendications 1 à 22, pour le nettoyage des peaux à tendance grasse ou acnéique.

27. Utilisation cosmétique de 0,01 à 0,05 % en poids de chlorure de N-(3-chloroallyl)-hexaminium et de 0,005 à 0,3 % en poids de parahydroxy-benzoate d'alkyle en C₁-C₄ par rapport au poids total de la composition, en tant qu'agent de lutte contre les micro-organismes dans une composition cosmétique contenant une phase aqueuse et destinée à imprégner un substrat insoluble dans l'eau, non tissé.

28. Procédé cosmétique de nettoyage et/ou de démaquillage de la peau et/ou des yeux, consistant à passer sur la peau et/ou les yeux, une lingette selon l'une quelconque des revendications 1 à 22.

## Patentansprüche

1. Tuch für die Haut, das (A) ein in Wasser unlösliches, nicht gewebtes Substrat und (B) eine Zusammensetzung umfasst, die zu dem Substrat gegeben wurde oder mit der das Substrat getränkt ist und die in einem physiologisch akzeptablen Medium eine wässrige Phase und 0,01 bis 0,05 Gew.-% N-(3-Chlorallyl)-hexaminiumchlorid, bezogen auf das Gesamtgewicht der Zusammensetzung, und 0,005 bis 03, Gew.-% C₁₋₄-Alkyl-para-hydroxybenzoat enthält.

2. Tuch nach Anspruch 1, **dadurch gekennzeichnet, dass** die Zusammensetzung 0,01 bis 0,03 Gew.-% N-(3-Chlorallyl)-hexaminiumchlorid, bezogen auf das Gesamtgewicht der Zusammensetzung, enthält.

3. Tuch nach Anspruch 1 oder 2, **dadurch gekennzeichnet, dass** die Zusammensetzung ferner mindestens ein Konservierungsmittel enthält, das unter den Salzen der Ethylendiamintetraessigsäure und deren Gemischen ausgewählt ist.

4. Tuch nach einem der vorhergehenden Ansprüche, **dadurch gekennzeichnet, dass** das C₁₋₄-Alkyl-para-hydroxybenzoat unter Methyl-para-hydroxybenzoat, Ethyl-para-hydroxybenzoat, Propyl-para-hydroxybenzoat und deren Gemischen ausgewählt ist.

5. , Tuch nach dem vorhergehenden Anspruch, **dadurch gekennzeichnet, dass** das para-Hydroxybenzoat das Methyl-para-hydroxybenzoat ist.

6. Tuch nach Anspruch 3, **dadurch gekennzeichnet, dass** das Ethylendiamintetraessigsäuresalz unter dem Dinatriumsalz von Ethylendiamintetraessigsäure, dem Tetranatrium salz von Ethylendiamintetraessigsäure und deren Gemischen ausgewählt ist.

7. Tuch nach einem der Ansprüche 3 oder 6, **dadurch gekennzeichnet, dass** die Zusammensetzung 0,01 bis 0,2 Gew.-% Ethylendiamintetraessigsäuresalz, bezogen auf das Gesamtgewicht der Zusammensetzung, enthält.

8. Tuch nach einem der vorhergehenden Ansprüche, **dadurch gekennzeichnet, dass** die Zusammensetzung eine Viskosität unter 100 mPa·s aufweist.

9. Tuch nach einem der vorhergehenden Ansprüche, **dadurch gekennzeichnet, dass** die Zusammensetzung in Form eines wässrigen oder wässrig-alkoholischen Lösung, als Emulsion, als Mikroemulsion, in Form eines wässrigen oder wässrig alkoholischen Gels oder als Vesikeldispersion vorliegt.

10. Tuch nach einem der vorhergehenden Ansprüche, **dadurch gekennzeichnet, dass** die Zusammensetzung als O/W-Emulsion vorliegt.

11. Tuch nach einem der vorhergehenden Ansprüche, **dadurch gekennzeichnet, dass** die Zusammensetzung in Form einer PIT-Emulsion vorliegt.

12. Tuch nach einem der vorhergehenden Ansprüche, dadurch gekennzeiehnet, dass die Zusammensetzung ferner einen Emulgator enthält, der unter den Polyolfettsäureestern; Estern einer Fettsäure und alkoxylierten Polyolen; Ethern von Fettalkoholen und Polyolen; Ethern von Fettalkoholen und alkoxylierten Polyolen; und deren Gemischen ausgewählt ist.

13. Tuch nach einem der vorhergehenden Ansprüche, **dadurch gekennzeichnet, dass** die Zusammensetzung ferner mindestens einen Zusatzstoff enthält, der unter den organischen Lösungsmit-
satoren, Schaumbildnern, Füllstoffen, Maskierungmitteln, Parfums, Filtern, etherischen Ölen, Farbstoffen, Pigmenten, Wirkstoffen und Lipidvesikeln ausgewählt ist.

14. Tuch nach dem vorhergehenden Anspruch, **dadurch gekennzeichnet, dass** der Wirkstoff unter den Wirkstoffen gegen Seborrhoe, antimikrobiellen Wirkstoffen und deren Gemischen ausgewählt ist.

15. Tuch nach dem vorhergehenden Anspruch, **dadurch gekennzeichnet, dass** der Wirkstoff gegen Seborrhoe unter Schwefel und schwefelhaltigen Derivaten, Benzoylperoxid, Zinkderivaten, Aluminiumchlorid, Selendisulfid, Panthenol, Niacinamid und deren Gemischen ausgewählt ist.

16. Tuch nach Anspruch 14 oder 15, **dadurch gekennzeichnet, dass** der antimikrobielle Wirkstoff unter 2,4,4'-Trichlor-2'-hydroxydiphenylether, 3,4,4'-Trichlorobanilid, Phenoxyethanol, Phenoxypropanol, Phenoxyisopropanol, Chlorhexidin und seinen Salzen, Octopirox, Zink-Pyrithion, Salicylsäure, n-Octanol-5-salicylsäure, Benzoylperoxid, 3-Hydroxybenzoesäure, Glykolsäure, Milchsäure, 4-Hydroxybenzoesäure, Acetylsalicylsäure, 2-Hydroxybutansäure, 2-Hydroxypentansäure, 2-Hydroxyhexansäure, Phytinsäure, N-Acetyl-L-cystein, Liponsäure, Azelainsäure, Arachidonsäure, Octoxyglycerin, Octanoylglycin, Caprylylglykol, 10-Hydroxy-2-decansäure und deren Gemischen ausgewählt ist.

17. Tuch nach einem der vorhergehenden Ansprüche, **dadurch gekennzeichnet, dass** die Zusammensetzung ferner mindestens einen schäumenden grenzflächenaktiven Stoff enthält.

18. Tuch nach dem vorhergehenden Anspruch, **dadurch gekennzeichnet, dass** der schäumende grenzflächenaktive Stoff unter den ethoxylierten, propoxylierten Blockpolymeren, Alkylpolyglucosiden; Alkylsulfaten, Alkylethersulfaten und deren Salzen; Alkylamidoslkylaminderivaten und deren Gemischen ausgewählt ist.

19. Tuch nach einem der vorhergehenden Ansprüche, **dadurch gekennzeichnet, dass** die Zusammensetzung keine 2-Pyrrolidon-5-carbonsäure enthält.

20. Tuch nach einem der vorhergehenden Ansprüche, **dadurch gekennzeichnet, dass** die Zusammensetzung einen pH-Wert von 6 bis 7, 5 aufweist.

21. Tuch nach einem der vorhergehenden Ansprüche, **dadurch gekennzeichnet, dass** die Zusammensetzung als Lotion oder als Milch vorliegt.

22. Tuch nach einem der vorhergehenden Ansprüche, **dadurch gekennzeichnet, dass** es 200 bis 1 000 Gew.-% Zusammensetzung, bezogen auf das Gewicht des Substrats, umfasst.

23. Tuch nach einem der vorhergehenden Ansprüche, **dadurch gekennzeichnet, dass** es sich um ein Tuch zur Pflege und/ oder zur Behandlung der Haut handelt.

24. Tuch nach einem der vorhergehenden Ansprüche, **dadurch gekennzeichnet, dass** es ein Tuch zur Reinigung und/oder zum Abschminken der Haut und/ oder der Augen ist.

25. Kosmetische Verwendung des Tuchs nach einem der Ansprüche 1 bis 22 zur Reinigung und/oder zum Abschminken der Haut und/oder der Augen.

26. Kosmetische Verwendung eines Tuchs nach einem der Ansprüche 1 bis 22 zur Reinigung von Haut mit Tendenz zu fettiger Haut oder Tendenz zu Akne.

27. Kosmetische Verwendung von 0,01 bis 0,05 Gew.-% N-(3-Chlorallyl)-hexaminiumchlorid und 0,005 bis 0,3 Gew.-% C₁₋₄-Alkyl-para-hydroxybenzoat, bezogen auf das Gesamtgewicht der Zusammensetzung, als Wirkstoff zur Bekämpfung von Mikroorganismen in einer kosmetischen Zusammensetzung, die eine wässrige Phase enthält und dazu vorgesehen ist, ein in Wasser unlösliches, nicht gewebtes Substrat zu tränken.

28. Kosmetisches Verfahren zur Reinigung und/oder zum Abschminken der Haut und/oder der Augen, das darin besteht, über die Haut und/oder die Augen ein Tuch nach einem der Ansprüche 1 bis 22 zu führen.

## Claims

1. Wipe for the skin comprising (A) a nonwoven, water-insoluble substrate and (B) a composition added to or impregnated onto the substrate, comprising, in a physiologically acceptable medium, an aqueous phase and from 0.01% to 0.05% by weight of N-(3-chloroallyl)hexaminium chloride relative to the total weight of the composition, and from 0.005% to 0.3% by weight of C₁-C₄ alkyl para-hydroxybenzoate.

2. Wipe according to Claim 1, **characterized in that** the composition comprises from 0.01% to 0.03% by weight of N-(3-chloroallyl)hexaminium chloride relative to the total weight of the composition.

3. Wipe according to Claim 1 or 2, **characterized in that** the composition also comprises at least one preserving agent chosen from ethylenediaminetetraacetic acid salts, and mixtures thereof.

4. Wipe according to any one of the preceding claims, **characterized in that** the C₁-C₄ alkyl para-hydroxybenzoate is chosen from methyl para-hydroxybenzoate, ethyl para-hydroxybenzoate and propyl para-hydroxybenzoate, and mixtures thereof.

5. Wipe according to the preceding claim, **characterized in that** the para-hydroxybenzoate is methyl para-hydroxybenzoate.

6. Wipe according to Claim 3, **characterized in that** the ethylenediaminetetraacetic acid salt is chosen from the disodium salt of ethylenediaminetetraacetic acid and the tetrasodium salt of ethylenediaminetetraacetic acid, and mixtures thereof.

7. Wipe according to either of Claims 3 and 6, **characterized in that** the composition comprises from 0.01% to 0.2% by weight of ethylenediaminetetraacetic acid salt relative to the total weight of the composition.

8. Wipe according to any one of the preceding claims, **characterized in that** the composition has a viscosity of less than 100 mPa.s.

9. Wipe according to any one of the preceding claims, **characterized in that** the composition is in the form of an aqueous or aqueous-alcoholic solution, an emulsion, a microemulsion, an aqueous or aqueous-alcoholic gel, or a vesicular dispersion.

10. Wipe according to any one of the preceding claims, **characterized in that** the composition is in the form of an O/W emulsion.

11. Wipe according to any one of the preceding claims, **characterized in that** the composition is in the form of a PIT emulsion.

12. Wipe according to any one of the preceding claims, **characterized in that** the composition also comprises an emulsifier chosen from fatty acid esters of polyols; oxyalkylenated fatty acid esters of polyols; fatty alcohol ethers of polyols; oxyalkylenated fatty alcohol ethers of polyols; and mixtures thereof.

13. Wipe according to any one of the preceding claims, **characterized in that** the composition also comprises at least one adjuvant chosen from organic solvents, solubilizing agents, thickeners and gelling agents, softeners, antioxidants, opacifiers, stabilizers, foaming agents, fillers, chelating agents, fragrances, screening agents, essential oils, dyestuffs, pigments, active agents and lipid vesicles.

14. Wipe according to the preceding claim, **characterized in that** the active agent is chosen from antiseborrhoeic active agents and antimicrobial active agents, and mixtures thereof.

15. Wipe according to the preceding claim, **characterized in that** the antiseborrhoeic active agent is chosen from sulphur and sulphur derivatives, benzoyl peroxide, zinc derivatives, aluminium chloride, selenium disulphide, panthenol and niacinamide, and mixtures thereof.

16. Wipe according to Claim 14 or 15, **characterized in that** the antimicrobial active agent is chosen from 2,4,4'-trichloro-2'-hydroxydiphenyl ether, 3,4,4'-trichlorobanilide, phenoxyethanol, phenoxypropanol, phenoxyisopropanol, chlorhexidine and its salts, octopirox, zinc pyrithione, salicylic acid, 5-n-octanoylsalicylic acid, benzoyl peroxide, 3-hydroxybenzoic acid, glycolic acid, lactic acid, 4-hydroxybenzoic acid, acetylsalicylic acid, 2-hydroxybutanoic acid, 2-hydroxypentanoic acid, 2-hydroxyhexanoic acid, phytic acid, N-acetyl-L-cysteine acid, lipoic acid, azelaic acid, arachidonic acid, octoxyglycerol, octanoylglycine, caprylyl glycol and 10-hydroxy-2-decanoic acid, and mixtures thereof.

17. Wipe according to any one of the preceding claims, **characterized in that** the composition also comprises at least one foaming surfactant.

18. Wipe according to the preceding claim, **characterized in that** the foaming surfactant is chosen from oxyethylenated oxypropylenated block polymers, alkylpolyglucosides; alkyl sulphates, alkyl ether sulphates and salts thereof; alkylamido alkylamine derivatives; and mixtures thereof.

19. Wipe according to any one of the preceding claims, **characterized in that** the composition is free from 2-pyrrolidone-5-carboxylic acid.

20. Wipe according to any one of the preceding claims, **characterized in that** the composition has a pH of from 6 to 7.5.

21. Wipe according to any one of the preceding claims, **characterized in that** the composition is in the form of a lotion or a milk.

22. Wipe according to any one of the preceding claims, **characterized in that** it comprises from 200% to 1000% by weight of composition relative to the weight of substrate.

23. Wipe according to any one of the preceding claims, **characterized in that** it is a skincare wipe and/or a skin treatment wipe.

24. Wipe according to any one of the preceding claims,
**characterized in that** it is a wipe for cleansing and/or removing makeup from the skin and/or the eyes.

25. Cosmetic use of the wipe according to any one of Claims 1 to 22, for cleansing and/or removing makeup from the skin and/or the eyes.

26. Cosmetic use of a wipe according to any one of Claims 1 to 22, for cleansing greasy skin or acneprone skin.

27. Cosmetic use of 0.01% to 0.05% by weight of N- (3-chloroallyl)hexaminium chloride and of 0.005% to 0.3% by weight of C₁-C₄ alkyl para-hydroxybenzoate relative to the total weight of the composition, as an agent for combating microorganisms in a cosmetic composition containing an aqueous phase and intended to impregnate a nonwoven water-insoluble substrate.

28. Cosmetic process for cleansing and/or removing makeup from the skin and/or the eyes, which consists in passing a wipe according to any one of Claims 1 to 22 over the skin and/or the eyes.
